Europäisches Patentamt

European Patent Office

Office européen des brevets

(11) Numéro de publication : **0 417 003 B1**

(12) **FASCICULE DE BREVET EUROPEEN**

(45) Date de publication du fascicule du brevet :
**29.06.94 Bulletin 94/26**

(51) Int. Cl.$^5$ : **A61K 31/565**

(21) Numéro de dépôt : **90402449.4**

(22) Date de dépôt : **06.09.90**

(54) **Utilisation de composés antiprogestomimétiques pour favoriser l'ovulation.**

(30) Priorité : **07.09.89 FR 8911699**

(43) Date de publication de la demande :
**13.03.91 Bulletin 91/11**

(45) Mention de la délivrance du brevet :
**29.06.94 Bulletin 94/26**

(84) Etats contractants désignés :
**AT BE CH DE DK FR GB IT LI LU NL SE**

(56) Documents cités :
**EP-A- 0 083 925**
**EP-A- 0 256 224**
**EP-A- 0 266 303**
**FR-A- 2 522 328**
**FR-A- 2 566 779**
**BIOLOGY OF REPRODUCTION, vol. 34, no. 3, avril 1986, pages 488-494; S. KANEKO et al.: "Changes in plasma progesterone, estradiol, follicle-stimulating hormone and luteinizing hormone during diestrus and ovulation in rats with 5-day estrous cycles: effect of antibody against progesterone"**

(56) Documents cités :
**NEURO-ENDOCRINOLOGY OF REPRODUCTION, PROCEEDINGS OF THE VIth REINIER DE GRAAF SYMPOSIUM, Nijmegen, 27-29 août 1987, vol. 751, pages 161-168, Elsevier Science Publishers B.V. (Biomedical Division); P. van der SCHOOT et al.: "The progesterone antagonist mifepristone, a tool for the study of the role of progesterone in regulating ovarian activity in rats"**
**AMERICAN JOURNAL OF OBSTETRICS AND GYNECOLOGY, vol. 159, no. 6, décembre 1988, pages 1584-1589, The C.V. Mosby Co., St. Louis, Missouri, US; S.I. ROH et al.: "The effects of progesterone antagonist RU 486 on mouse oocyte maturation, ovulation, fertilization, and cleavage"**

(73) Titulaire : **ROUSSEL-UCLAF**
**35, Boulevard des Invalides**
**F-75007 Paris (FR)**

(72) Inventeur : **Grandadam, Jean André**
**56, Avenue Gabriel Péri**
**F-94100 Saint Maur Des Fosses (FR)**

(74) Mandataire : **Tonnellier, Marie-José et al**
**111, route de Noisy**
**B.P. no 9**
**F-93230 Romainville (FR)**

## Description

La présente invention concerne une nouvelle utilisation de certains composés anti-progestomimétiques.

L'invention a pour objet l'utilisation de composés anti-progestomimétiques pour la fabrication de compositions pharmaceutiques destinées à favoriser l'ovulation.

L'utilisation selon l'invention a lieu aussi bien en médecine humaine qu'en médecine vétérinaire.

Le composé anti-progestomimétique est choisi parmi les composés dont les noms suivent :

- le 17béta-hydroxy 11béta-(4-diméthylaminophényl) 17alpha-(prop-1-ynyl) estra 4,9-dièn-3-one (appelé ci-après produit A);
- le (Z) 11béta-[4-(diméthylamino) phényl] 17béta-hydroxy 17alpha-(1-propényl) estra 4,9-dièn 3-one (appelé ci-après produit B).

Les composés utilisés peuvent être préparés comme indiqué dans les brevets européens 0 057 115 et 0 262 188 ainsi que dans les brevets 2 566 779 et 2 625 505.

L'administration de composés antiprogestomimétiques a lieu à la suite d'un traitement de 3-oxo 17alpha-allyl 17béta-hydroxy estra 4,9,11-triène (appelé ci-après produit C) décrit dans le brevet spécial de médicament 5183 M.

Une des formes d'utilisation préférée de l'invention a lieu en médecine vétérinaire, à la suite d'un traitement de synchronisation d'oestrus.

Un traitement de synchronisation d'oestrus ne semble pas avoir en lui-même d'effet positif sur la ponte ovulaire.

L'utilisation, objet de la présente demande de brevet, présente l'avantage d'augmenter ou de favoriser la ponte ovulaire, ce qui est naturellement d'un grand intérêt dans les élevages et notamment dans les élevages industriels.

On utilise comme traitement de synchronisation d'oestrus un traitement de 20 à 60 mg par jour et par animal pendant 10 à 30 jours de produit C administré par voie buccale dans l'alimentation, par exemple, un traitement de 15 à 20 jours de 35 à 45 mg de produit C.

On administre ensuite le ou les produits anti-progestomimétiques à la suite d'un traitement de synchronisation de préférence en une seule injection, par exemple, une seule injection des produits A ou B à une dose comprise entre 0,5 et 5000 mg par animal, et de préférence à une dose comprise entre 500 et 5000 mg par animal.

L'invention a plus particulièrement pour objet l'utilisation caractérisée en ce que l'administration du ou des produits a lieu chez les animaux d'élevage.

Par animaux d'élevage, on entend les bovins, les ovins, les caprins, les porcins, les chevaux ou encore les chiens et les chats.

L'invention a plus particulièrement pour objet l'utilisation caractérisée en ce que l'administration a lieu chez la vache et notamment chez la génisse.

L'utilisation selon l'invention s'étend également au domaine de la médecine humaine, dans le traitement des troubles de l'ovulation, dysovulation et même anovulation.

L'utilisation selon l'invention permet donc de lutter contre certaines formes de stérilité.

L'essai biologique, décrit ci-après, illustre l'invention.

Essai biologique

On utilise 30 génises de race à viande, 15 charolaises et 15 Blanc, Bleu, Belges ; on détecte les vaches cyclées par le dosage de progestérone plasmatique effectué 2 fois à 10 jours d'intervalle.

On fait l'essai sur les 8 génisses cyclées.

Les animaux reçoivent un traitement de 3-oxo 17alpha-allyl 17beta-hydroxy estra 4,9,11-triène (produit C) agissant en tant que synchroniseur d'oestrus.

Le produit est administré per os à raison de 40 mg par animal pendant 18 jours.

Le jour suivant la dernière distribution du produit, on administre par injection sous cutanée une dose de 1 mg de produit A par kg du poids d'animal.

On dose selon les méthodes classiques :

- La progestérone plasmatique ; son taux augmente de 1 à 10 ng/ml pendant la phase lutéale. Son dosage se fait par radio-immunologie après extraction plasmatique à l'éther.
- L'hormone luteinisante ou LH ; son taux augmente de 5 à 30 ng/ml 6 à 12 heures avant l'ovulation. Son dosage se fait par radio-immunologie directement sur le plasma.
- L'oestradiol ; son taux augmente de 2 à 20 ng/ml durant la phase oestrale.

Ce protocole de l'essai est résumé sur le tableau suivant :

## CALENDRIER DES INTERVENTIONS

JOURS    INTERVENTIONS


J-13    : prélèvement sanguin 1 tube 10 ml | vérification
          sur héparine                     | de la cyclicité :
J-3     : prélèvement sanguin 1 tube 10 ml | dosage proges-
          sur héparine                     | térone


J-2     : - résultats dosage progestérone
          - choix de 8 génisses


## Produit C


JO      : prélèvement avant traitement
          2 tubes sur héparine
          dosage progestérone et produit C
          début distribution produit C per os

(suite Produit C)

J1      : prélèvement 2 tubes sur héparine
             suite distribution produit C

J8      : prélèvement 2 tubes sur héparine

J15     : prélèvement 2 tubes sur héparine

J17     : Dernière distribution de produit C

<u>Produit A</u>

          : Injections de produit A

J18     : - 17 H : prélèvement 2 tubes sur héparine : dosages
                      oestradiol - LH - progestérone

J19     : -  8 H
             - 14 H   prélèvement 2 tubes sur héparine : dosages
             - 20 H   oestradiol - LH - progestérone

J20     : -  8 H
             - 14 H   prélèvement 2 tubes sur héparine : dosages
             - 20 H   oestradiol - LH - progestérone

J21     : -  8 H
             - 14 H   prélèvement 2 tubes sur héparine : dosages
             - 20 H   oestradiol - LH - progestérone

J22     : -  8 H   prélèvement 2 tubes sur héparine : dosages
             - 14 H   oestradiol - LH - progestérone

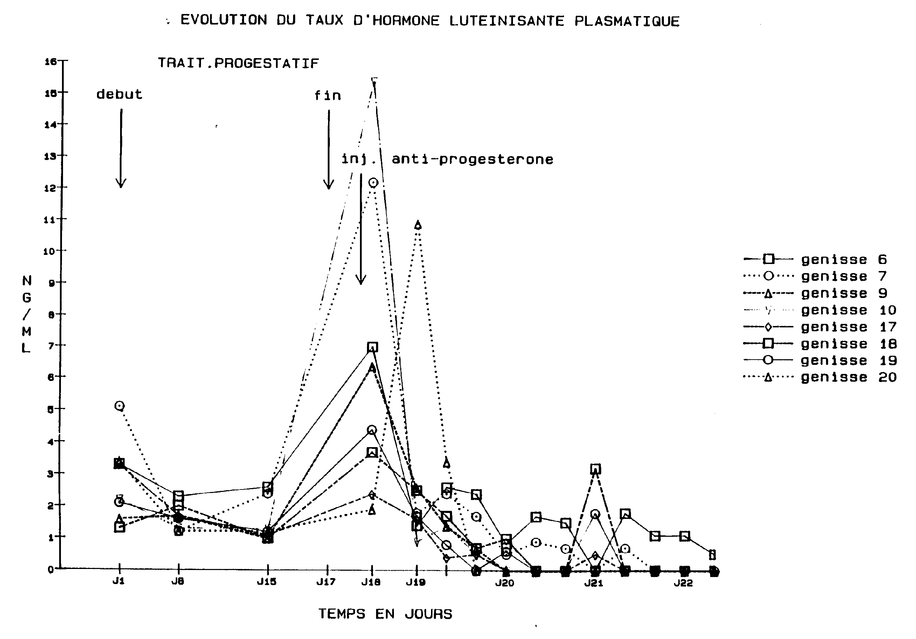

Les résultats représentés sur le diagramme de la planche 1/1 indique que toutes les génisses sont venues en chaleur dans un délai très court. Le taux de LH est monté extrêmement rapidement.

**Revendications**

   **1.**    Utilisation du 17béta-hydroxy 11béta-(4-diméthyl aminophényl) 17alpha-(prop-1-ynyl) estra 4,9-dien-3-one ou du (Z) 17béta-hydroxy 11béta-(4-diméthyl aminophényl) 17alpha-(prop-1-enyl) estra-4,9-dien 3-one pour la fabrication de compositions pharmaceutiques destinées à favoriser l'ovulation, à utiliser à la suite d'un traitement de 3-oxo 17alpha allyl 17béta-hydroxy-estra 4,9,11-triene.

2. Utilisation selon la revendication 1 caractérisée en ce que l'administration a lieu chez les animaux d'élevage.

3. Utilisation selon la revendication 2 caractérisée en ce que l'administration a lieu chez la génisse.

**Patentansprüche**

1. Verwendung von 17β-Hydroxy-11β-(4-dimethylaminophenyl)-17α-(1-propinyl)-estra-4,9-dien-3-on oder von (Z)17β-Hydroxy-11β-(4-dimethylaminophenyl)-17α-(1-propenyl)-estra-4,9-dien-3-on zur Herstellung von pharmazeutischen Zusammensetzungen für die Stimulierung der Ovulation, angewendet nach einer Behandlung mit 3-Oxo-17α-allyl-17β-hydroxy-estra-4,9,11-trien.

2. Verwendung nach Anspruch 1, dadurch gekennzeichnet, daß die Verabreichung bei Zuchttieren stattfindet.

3. Verwendung nach Anspruch 2, dadurch gekennzeichnet, daß die Verabreichung bei Färsen stattfindet.

**Claims**

1. Use of 17beta-hydroxy 11beta-(4-dimethyl aminophenyl) 17alpha-(prop-1-ynyl) estra 4,9-dien-3-one or of (Z) 17beta-hydroxy 11beta-(4-dimethyl aminophenyl) 17alpha-(prop-1-enyl) estra-4,9-dien 3-one for the manufacture of pharmaceutical compositions intended for stimulating ovulation, to be used following treatment with 3-oxo 17alpha allyl 17beta-hydroxy-estra 4,9,11-triene.

2. Use according to claim 1 characterized in that the administration takes place in farm animals.

3. Use according to claim 2 characterized in that the administration takes place in heifers.

EVOLUTION DU TAUX D'HORMONE LUTEINISANTE PLASMATIQUE

TRAIT.PROGESTATIF

debut

fin

inj. anti-progesterone

N G / M L

TEMPS EN JOURS

genisse 6
genisse 7
genisse 9
genisse 10
genisse 17
genisse 18
genisse 19
genisse 20

EP 0 417 003 B1

6